# EUROPÄISCHE PATENTSCHRIFT

(11) **EP 1 071 757 B1**
(45) Veröffentlichungstag und Bekanntmachung des Hinweises auf die Patenterteilung: **09.11.2005**
(21) Anmeldenummer: 99907223.4
(22) Anmeldetag: 19.03.1999
(51) Int. Cl.: C12N 15/00

(54) **KOMBINATION VON GENEN ZUR REGULIERUNG DER BLÜHINDUKTION BEI NUTZ- UND ZIERPFLANZEN**
COMBINATION OF GENES FOR REGULATING FLOWERING INDUCTION IN USEFUL AND ORNAMENTAL PLANTS
ASSOCIATION DE GENES POUR LA REGULATION DE L'INDUCTION DE LA FLORAISON CHEZ LES PLANTES CULTIVEES ET LES PLANTES D'ORNEMENT

(30) Priorität: 19.03.1998 CH 65798
(43) Veröffentlichungstag der Anmeldung: 31.01.2001
(73) Patentinhaber: Syngenta Participations AG, 4058 Basel (CH)
(72) Erfinder: GLEISSNER, Roland, CH-8048 Zürich (CH); MELZER, Siegbert, CH-8353 Elgg (CH); KURZ, Birgit, CH-8352 Räterschen (CH); APEL, Klaus, D-78315 Radolfzell (DE)
(74) Vertreter: Bastian, Werner Maria
(86) Internationale Anmeldenummer: PCT/CH1999/000122
(87) Internationale Veröffentlichungsnummer: WO 1999/047654

(56) Entgegenhaltungen:
- WO-A-96/11566
- WO-A-97/25433
- KANIA T, ET AL.: "FPF1 Promotes Flowering in Arabidopsis" THE PLANT CELL, Bd. 9, Nr. 8, August 1997 (1997-08), Seiten 1327-1338, XP002112935 in der Anmeldung erwähnt
- MENZEL G ET AL: "Identification of two MADS box genes that are expressed in the apical meristem of the long-day plant Sinapis alba in transition to flowering" PLANT JOURNAL, Bd. 9, Nr. 3, 1. März 1996 (1996-03-01), Seiten 399-408, XP002082195 ISSN: 0960-7412
- LEVY Y, DEAN C: "control of flowering time" CURRENT OPINION IN PLANT BIOLOGY, Bd. 1, Nr. 1, Februar 1998 (1998-02), Seiten 49-54, XP002112936

## Beschreibung

Die vorliegende Erfindung bezieht sich auf die Regulierung des Blühzeitpunkts bei Nutz- und Zierpflanzen.

Alle bisher bekannten Verfahren zur Regulation der Blütenbildung basieren auf der Überexpression eines einzelnen Gens. Da die Induktion der Blütenbildung, wie im folgenden beschrieben, von einem Netzwerk von daran beteiligten Genen reguliert wird, von denen viele noch nicht kloniert und charakterisiert wurden, ist dieser Weg nicht optimal für eine ausgewogene Entwicklung der Pflanzen.

Der Übergang vom vegetativen Wachstum zur Blütenbildung ist ein deutlich sichtbarer Wechsel zu einem neuen Entwicklungsprogramm einer Pflanze. Er zeigt eine Änderung der Funktion des apikalen Meristems, das von der Blattbildung zur Bildung von Blüten übergeht. Dieser morphogenetische Wechsel wird entweder durch endogene Faktoren kontrolliert, indem das genetische Programm zur Blütenbildung nach einer bestimmten Zeit des vegetativen Wachstums "angestellt" wird, d.h. nachdem eine definierte Anzahl von Blättern produziert wurde, oder aber durch verschiedene Umweltbedingungen. Die wichtigsten und am eingehendsten untersuchten Umweltbedingungen sind niedrige Temperaturen (Vernalisation) und die Tageslichtlänge (Photoperiode). In Gewächshäusern können diese Umweltbedingungen angepasst werden, um eine optimale Anzucht von Pflanzen zu gewährleisten beziehungsweise um beim Übergang vom vegetativen Wachstum zur Blütenbildung einen grösstmöglichen Reproduktionserfolg zu erzielen. Dies erfordert jedoch einen beträchtlichen Einsatz an nicht erneuerbaren Energien. Unter Feldbedingungen ist dies nicht ohne weiteres möglich. Es ist deshalb ein Ziel der klassischen Pflanzenzüchtung, Sorten mit einem definierten Blühzeitpunkt zu selektionieren. Im Fall von frühblühenden Sorten wäre es dann möglich, wichtige Kulturpflanzen auch in Regionen anzubauen, in denen sie natürlicherweise nicht zur vollständigen Reife gelangen. Die Selektion frühblühender Sorten durch die klassische Züchtung ist jedoch sehr zeitintensiv.

Seit bekannt ist, dass die Lichtperiode ein wichtiger Faktor bei der Regulierung der Blühzeit ist, haben Pfropf- und Defoliationsexperimente Hinweise ergeben, dass ein bislang noch unbekannter Blühstimulus in den Blättern von Pflanzen produziert wird, wenn sie einer kritischen Tageslänge ausgesetzt sind. Dieses Signal wird von den Blättern über das Phloem zu dem apikalen Meristem transportiert. Wenn dieser Blühstimulus ein apikales Meristem erreicht hat, das bereit ist, auf dieses Signal zu reagieren, wird die Blütenbildung initiiert.

Blühzeitmutanten von *Arabidopsis thaliana,* die später oder früher blühen als die entsprechenden Wildtyppflanzen, spielen eine wichtige Rolle bei der Aufklärung der Induktionsereignisse in den Blättern und des Signalübertragungsweges von den Blättern zum Meristem. Es wurden bei *Arabidopsis* bisher 13 verschiedenene Gene mit Hilfe von Spätblühmutanten identifiziert, die eine Rolle bei der Induktion der Blütenbildung spielen. Diese Gene konnten durch genetische Untersuchungen in drei parallele Signaltransduktionswege eingeordnet werden (Koornneef, M. et al., Mol. Gen. Genet.229, 57-66, 1991). Die beiden zuerst klonierten Gene, die bei der Bestimmung des Blühzeitpunktes von *Arabidopsis* eine Rolle spielen, codieren regulatorische Proteine, die konstitutiv exprimiert werden. *LUMINIDEPENDENS (LD)* scheint die Lichtperzeption zu beeinflussen (Lee et al., Plant Cell 6, 75-83, 1994) und *CONSTANS (CO)* ist für die Induktion der Blütenbildung unter Langtagbedingungen notwendig (Putterill et al., Cell 80, 847-857, 1995). Ein weiteres Gen, das den Übergang zur Blütenbildung reguliert, ist *FCA.* Dieses Gen wurde kloniert und es konnte gezeigt werden, dass das codierte Protein zwei RNA-Bindungsstellen und eine Protein-Interaktionsdomäne besitzt. Es wird deshalb angenommen, dass *FCA* in die Regulation der Transkriptmaturation von Genen eingreift, die bei der Blühinduktion eine Rolle spielen (Macknight et al., Cell 89, 737-745, 1997).

In den letzten Jahren wurden bemerkenswerte Fortschritte bezüglich des Verständnisses der Regulierung der Blütenorganogenese gemacht. Genetische und molekulare Untersuchungen der Blütenentwicklung in *Antirrhinum majus* und *Arabidopsis* haben zur Isolierung von Identitätsgenen des Blütenmeristems und der Blütenorgane geführt, die die Blütenbildung kontrollieren (Coen, E.S. und Meyerowitz, E.M., Nature 353, 31-37, 1991; Weigel, D. und Meyerowitz, E.M., Science 261, 1723-1726, 1993). Alle bis auf zwei dieser Gene codieren Genprodukte mit einer aminoterminalen DNA-Bindungsdomäne, die Homologien zu den DNA-Bindungsdomänen der Transkriptionsfaktoren *MCM1* der Hefe und *SRF* der Säugetiere aufweisen. Diese Domäne wurde als *MADS* Box bezeichnet, eine Abkürzung der Namen der zuerst klonierten Gene (Schwarz-Sommer et al., Science 250, 931-936, 1990). Es konnte nachgewiesen werden, dass die Identitätsgene der Blütenorgane zum Teil durch Genprodukte der Identitätsgene des Blütenmeristems wie *FLORICAULA (FLO)* in *Antirrhinum* (Hantke et al., Development 121, 27-35, 1995) und in *Arabidopsis* durch das *FLO*-Homolog *LEAFY (LFY)* und das *MADS* Box-Gen *APETALA1 (AP1)* (Weigel, D. und Meyerowitz, E.M., Science 261, 1723-1726, 1993) reguliert werden. Darüberhinaus konnte gezeigt werden, dass *TERMINAL FLOWER1 (TFL1)*, ein Gen, das für die Regulierung der Blütenmeristembildung und die Aufrechterhaltung des Infloreszenzmeristems verantwortlich ist, mit *LFY* und *AP1* (Gustafson-Brown, C. et al., Cell 76, 131-143, 1994; Shannon, S. und Meeks-Wagner, D.R., Plant Cell 5, 639-655, 1993; Weigel, D. et al., Cell 69, 843-859, 1992) und dass *CO* mit *LFY* interagiert (Putterill, J. et al., supra).

Ebenfalls nachgewiesen wurde, dass die konstitutive Expression von *LFY, AP1* und *CO* zu einer vorgezogenen Blütenbildung bei *Ara - bidopsis thaliana* führt (Weigel, D. and Nilsson, O., Nature 377, 495-500, 1995; Mandel, M.A. und Yanofsky, M.F., Nature 377, 522-544, 1995; Simon, R. et al., Nature 384, 59-62, 1996). Die ektopische Expression dieser Gene unter der Kontrolle des Cauliflower-Mosaikvirus (CaMV) 35S Promotors führt jedoch auch zu pleiotropen Effekten, die den Samenertrag stark beeinträchtigen. So führen alle drei genannten Gene *(LFY, AP1* und *CO)* zu einer Anlage von terminalen, fusionierten Blüten, die einen weiteren Blütenansatz in der Infloreszenz von *Arabidopsis* unterdrücken. Zur Ausbildung einer geschlossenen Infloreszenz mit einer vorzeitigen terminalen Blüte kommt es sonst in *Arabidopsis* nur nach Mutationen im Gen *TERMINAL FLOWER1* (*TFL1*), das normalerweise nach der Induktion der Blütenbildung kurz unterhalb des Infloreszenzmeristems angeschaltet wird (Bradley et al., Science 275, 80-83, 1997). Die ektopische Expression der regulatorischen Gene, die an der Blütenbildung beteiligt sind, scheinen *TFL1* zu reprimieren. Bei der *LFY*-Überexpression kommt es ausserdem zur Ausbildung von Blüten in den Blattachseln von transgenen Arabidopsispflanzen, die jeweils Schoten mit einem sehr reduzierten samenansatz entwickeln.

Weitere Gene, die den Blühzeitpunkt beinflussen, sind *OsMADS1* (Chung, Y.-Y. et al., Plant Molecular Biology 26, 657-665, 1994), das bei einer konstitutiven Expression in transgenen Tabakpflanzen zu Zwergwuchs und gestauchten Infloreszenzen führt, sowie *SPL3* (Cardon, G.H. et al., Plant Journal 12, 367-377, 1997).

Die Gene *MADSA* (Genbank Accession Nr. U25696) und *MADSB* (Genbank Accession Nr. U25695) (Menzel, S. et al., Plant Journal 9, 399-408, 1996) und *FPF1* (EMBL Accession Nr. Y11987 für *SaFPF1* und Y11988 für *ATFPF1*) (Kania, T. et al., Plant Cell 9, 1327-1338, 1997) wurden ursprünglich aus dem Senf *(Sinapis alba*) isoliert. Es konnte gezeigt werden, dass diese Gene vor *LFY* und *AP1* im apikalen Meristem nach der Induktion der Blütenbildung induziert werden.

*MADSA* und *MADSB* wurden unter Verwendung der MADS Box codierenden Region des Blütenorganidentitätsgens *AGAMOUS (AG)* identifiziert (Menzel et al., supra). Die beiden Gene werden während der Übergangsphase vom vegetativen Wachstum zur Blütenbildung in apikalen Meristemen von *Sinapis* alba und *Arabidopsis thaliana* exprimiert. RNA-Blot-Analysen haben bestätigt, dass die Anzahl der Transkripte der beiden Gene kurz nach der Induktion der Blüte drastisch erhöht wird und dass beide Gene früher als die MADS Box-Gene *AP1* und *AG* exprimiert werden. *In situ*-Hybridisierungen haben gezeigt, dass sich die Expression der Gene auf das apikale Meristem der induzierten Pflanzen während der frühen Phasen der reproduktiven Entwicklung beschränkt. Die Expression von *MADSA* ist zuerst im Zentrum des Meristems nachweisbar. In dieser Region können die frühesten Änderungen eines aktivierten Meristems durch klassische physiologische Verfahren nachgewiesen werden. *MADSA* könnte daher eine wichtige Funktion während des Übergangs vom vegetativen Wachstum zur Blütenbildung besitzen. Das zu *MADSB* homologe *Arabidopsis* Gen wurde auch als *AGL8* von Mandel und Yanofsky (Plant Cell, 9, 1763-1771, 1995) beschrieben (Genbank accession number U33473), während eine zu *MADSA* homologe Sequenz als EST (expressed sequence tag) (Newmann et al., Plant Physiol. 106, 1241-1255, 1994) aus *Arabidopsis* isoliert wurde (Genbank Accession Nr. H36826).

In einer weiteren Untersuchung wurde das Gen *Flowering Promoting Factorl* (*FPF1*) charakterisiert (Kania et al., supra), das im apikalen Meristem sofort nach der photoperiodischen Induktion der Blüte in den Langtag-Pflanzen *Sinapis alba* und *Arabidopsis thaliana* exprimiert wird. In frühen Übergangsstadien ist eine Expression von *FPF1* nur in der peripheren Zone des apikaler. Meristems nachweisbar. Später kann sie jedoch auch in Blütenmeristemen und axillären Meristemen nachgewiesen werden, die sekundäre Infloreszenzen bilden. Das *FPF1*-Gen codiert ein 12,6 kDa grosses Protein, das keinerlei Homologie zu irgendeinem bereits identifizierten Protein mit bekannter Funktion aufweist. Eine konstitutive Expression des Gens in *Arabidopsis* unter Kontrolle des CaMV35S-Promotors resultierte in einem dominant vererbbaren Merkmal einer frühen Blütenbildung sowohl unter Kurz- als auch Langtagbedingungen. Behandlungen mit Gibberellin (GA) und Paclobutrazol, einem Inhibitor der GA-Synthese, haben gezeigt, dass *FPF1* an einem GA-abhängigen Signalweg beteiligt ist und eine GA-Antwort in apikalen Meristemen während des Übergangs zur Blüte moduliert.

Die drei bereits charakterisierten Gene *MADSA, MADSB* und *FPF1* führen bei einer konstitutiven Expression zu einer vorgezogenen Blütenbildung in *Arabidopsis.* Die transgenen Pflanzen, die *MADSA* oder *FPF1* überexprimieren, zeigen dabei einen völlig normaler. Blüten- und Samenansatz. Bei *35S::AtMADSB* Linien, in denen das Transgen stark exprimiert wird, kommt es gelegentlich auch zu einer Anlage von fusionierten terminalen Blüten.

Aufgabe der vorliegenden Erfindung ist es, den Blühzeitpunkt bei Nutz- und Zierpflanzen unter Berücksichtigung einer ausgewogenen Entwicklung der Planzen zu regulieren.

Diese Aufgabe wird erfindungsgemäss durch Überexpression der kombinierten Gene *MADSA* und *FPF1* oder *MADSB* und *FPF1* gelöst, die durch den Cauliflower Mosaik Virus (CaMV) 35S Promotor in der ganzen Pflanze einschliesslich des apikalen Meristems aktiviert werden und damit eine vorzeitige Blütenbildung induzieren, ohne dabei den Ertrag und die Wüchsigkeit der Pflanzen zu beeinträchtigen.

Durch konstitutive Expression der drei Gene *MADSA, MADSB* und *FPF1* und deren Kombination ist eine Regulation der Blütenbildung unter Aufrechterhaltung der Produktivität möglich. Die Kombination der Gene kann mittels Vektoren erfolgen, die mehrere Gene unter der Kontrolle von verschiedenen Promotoren besitzen, oder durch Fusionsproteine, bei denen die wirksamen Domänen der einzelnen Proteine unter der Kontrolle eines einzelnen Promotors stehen.

Da alle drei ektopisch exprimierten Gene (*MADSA*, *MADSB* und *FPF1*) zu einer vorgezogenen Blütenbildung führen, wurde zunächst untersucht, ob die Wirkungen der drei Gene sich in Pflanzen, die zwei der Gene konstitutiv exprimieren, ergänzen. Für erste Untersuchungen wurden Kreuzungen zwischen Pflanzenlinien durchgeführt, die die verschiedenen Gene konstitutiv exprimieren. Neben *MADSA, MADSB* und *FPF1* wurden hierzu auch die Blühmeristemidentitätsgene *LFY* und *AP1* mit in die Untersuchungen aufgenommen.

Transgene *35S::LFY* Pflanzen bilden im Gegensatz zu Wildtyppflanzen Blüten bereits in den Achseln der Rosettenblätter. Die Anzahl der Rosettenblätter wird dagegen nicht reduziert. Die Anlage von Blüten am apikalen Meristem von *Arabidopsis* ist in Wildtyppflanzen gekoppelt mit einer Internodienelongation (sogenanntes Bolting) der Hauptachse (Hempel und Feldman, Planta 192, 276-286, 1994). In *35S::LFY* Pflanzen finden wir eine Blütenbildung ohne eine vorausgehende Streckung der Hauptachse. Da *35S::FPF1* Pflanzen vor der Blütenbildung eine vorzeitige Strekkung der Hauptachse zeigen, ist es naheliegend, dass eine konstitutive Expression von *LFY* nicht zu einer Aktivierung von *FPF1* führt. Nach einer Kreuzung von transgenen *355::LFY* Pflanzen mit transgenen *35S::FPF1* Pflanzen zeigen die Nachkommen, die beide Gene konstitutiv überexprimieren, wieder ein koordiniertes Blühen und Bolten. Die Anzahl der Rosettenblätter in den *35S::LFY-35S::FPF1*-Pflanzen ist dabei sowohl unter Lang- als auch unter Kurztagbedingungen im Vergleich zu *35S::FPF1*-Pflanzen deutlich reduziert.

Es wurde gezeigt, dass die konstitutive Expression von *AP1* zu einem von der Photoperiode unabhängigem Blühen führt (Mandel und Yanofsky, supra). Die schwache Expression von *AP1* führt dagegen zu einer Reduktion der vegetativen Phase unter Langtagbedingungen, hat aber kaum Auswirkungen unter Kurztagbedingungen, d.h die Pflanzen blühen im Kurztag nur unwesentlich früher als Wildtyppflanzen. Wird in eine schwachexprimierende 35S::*AP1*-Linie 35S::*FPF1* hineingekreuzt, dann blühen die Nachkommen, die beide Gene konstitutiv exprimieren, unter Kurztagbedingungen genauso schnell wie unter Langtagbedingungen. Der Einfluss der Photoperiode auf die Blütenbildung wurde damit wieder aufgehoben. Die beobachteten Veränderungen des Blühzeitpunktes sind hierbei nicht additiv, sondern es werden synergistische Effekte beobachtet. Dies kann mit einer erhöhten Kompetenz der transgenen 35S::*FPF1*-Pflanzen für die Wirkung des Blühmeristemidentitätsgenes *AP1* erklärt werden.

Auch nach Kreuzungen von transgenen 35S::*FPF1*-Pflanzen mit 35S::*MADSA-* und 35S::*MADSB*-Pflanzen zeigte sich, dass die NachKommen noch früher blühen, wenn *FPF1* und eines der anderen Gene gleichzeitig überexprimiert werden. Werden *MADSA* und *MADSB* gleichzeitig überexprimiert, dann blühen die Nachkommen jedoch nicht früher als die jeweiligen Elternpflanzen. Dies deutet darauf hin, dass diese beiden Gene im gleichen Signaltransduktionsweg aktiv sind.

Es konnte gezeigt werden, dass Pflanzen die *FPF1* konstitutiv exprimieren, kompetenter für die Blütenbildung werden, d.h. sie reagieren empfindlicher auf die zusätzliche Expression der Blühmeristemidentitätsgene *LFY* und *AP1*, sowie auf die Expression von *MADSA* und *MADSB.* Da die drei Gene *FPF1*, *MADSA* und *MADSB* bei einer Überexpression mit einer moderaten Transkriptmenge die Fertilität der Pflanzen sowie deren Vitalität nicht einschränken und der beobachtete Einfluss auf die Blütenbildung additiv ist, sind hiermit Voraussetzungen geschaffen, die die Regulation des Blühzeitpunktes in Nutz- und Zierpflanzen in einem bisher nicht bekannten Ausmass ermöglichen. Als Beispiele für solche Nutzpflanzen sind unter anderen Pflanzen der Gattungen Triticum, Oryza, Zea, Hordeum, Sorghum, Avena, Secale, Lolium, Festuca, Lotus, Medicago, Glycine, Brassica, Solanum, Beta, sowie Gemüse oder Früchte produzierende Pflanzen und angiosperme Bäume zu nennen.

Für die Kombination der Gene ergeben sich verschiedene Möglichkeiten. Die Gene können auf einem Transformationsvektor kombiniert und von verschiedenen Promotoren reguliert werden. Die Verwendung von verschiedenen Promotoren ist wichtig, da beobachtet wurde, dass Gene, die von identischen Promotoren in transgenen Pflanzen reguliert werden, teilweise durch Mechanismen die man unter dem Oberbegriff "Cosupression" zusammenfasst, stillgelegt werden können (Matzke et al., Plant Journal 9, 183-194, 1996). Die Gene können auch als Fusionsproteine gemeinsam unter der Kontrolle eines einzelnen Promotors stehen. Die verschiedenen Möglichkeiten der Kombination werden in den Beispielen dargelegt.

Eine weitere Aufgabe der vorliegenden Erfindung ist es, transgene Pflanzen zur Verfügung zu stellen, die *MADSA* und *MADSB* in Antisense-Orientierung exprimieren. Es konnte gezeigt werden, dass 35S::*ASMADSA* und 35S::*ASMADSB* deutlich später blühen, als entsprechende Kontrollpflanzen. Nach einer Kreuzung von *MADSA* und *MADSB* Antisenselinien wurde in Pflanzen, die beide Antisensekonstrukte exprimieren, ein noch späteres Blühen beobachtet. Die Verzögerung der Blütenbildung korrelierte dabei direkt mit der Stärke der Expression der Antisensekonstrukte. Da die Überexpression von *FPF1* die Kompetenz der Pflanzen für die Blütenbildung erhöht, führt umgekehrt die Unterdrückung der *FPF1* Expression auch zu einer Verringerung der Kompetenz für die Blütenbildung. So konnten transgene Linien selektiert werden, die *FPF1* in Antisenseorientierung exprimieren und dadurch deutlich später blühen als entsprechende Kontrollpflanzen. Eine Selektion von geeigneten Linien, die verschiedene Antisensekonstrukte exprimieren, ermöglicht daher eine vollständige Unterdrückung der Blütenbildung.

Bei Pflanzen, von denen man in der Regel nur die vegetativen Teile erntet, können Antisense-Konstrukte verwendet werden, um eine ungewollte Blütenbildung zu verhindern. Dies spielt eine grosse Rolle bei Zuckerrüben, die Zucker nur im vegetativen Zustand in die Rüben einlagern. Beim Beginn der Blütenbildung wird dieser Zucker wieder mobilisiert und zum Aufbau der Infloreszenz verwendet. Dadurch kommt es zu nicht unerheblichen Ernteverlusten. Da für den Zuckerrübenanbau Hybridsaatgut ausgesät wird, muss sichergestellt sein, dass die Elternpflanzen noch blühen, um das Saatgut zu produzieren. Hier bietet sich eine Strategie an, bei der beide Elternteile mit verschiedenen Konstrukten transformiert werden, die für sich alleine zu keiner nennenswerten Reduktion der Blütenbildung führen. Erst das Zusammenwirken beider Konstrukte in den Hybridpflanzen führt dann zur Unterdrückung der ungewünschten Blütenbildung, die zu Ertragsverlusten führen würde. Eine weitere Möglichkeit die Expression nur in den Hybridpflanzen zuzulassen, bieten induzierbare Promotoren oder Promotoren die erst durch einen Aktivator aus einer der Elternpflanzen aktiv werden, wie es von Moore, I. et al., Proc. Natl. Acad. Sci. USA 95, 376-381, 1998 beschrieben worden ist.

Die vorliegende Erfindung wird durch die nachfolgenden Beispiele illustriert:

### Beispiel I

### Herstellung transgener Arabidopsis-Linien

### 1.1. Klonierung der Gene

Die Klonierung von *MADSA* und *MADSB* erfolgte wie in Menzel et al., Plant Journal 9, 399-408, 1996, beschrieben. *FPF1* wurde gemäss dem in Kania et al., Plant Cell 9, 1327-1338 beschriebenen Verfahren kloniert.

### 1.2. Überexpression der Gene

### FPF1:

Die Überexpression von *FPF1* wurde gemäss dem in der WO 97/25433 beschriebenen Verfahren durchgeführt.

### Sense-Konstrukte von MADSA und MADSB:

Zur Überexpression der *MADSA* und *MADSB* cDNAs aus Senf und *Arabidopsis* wurden die codierenden Bereiche der cDNAs durch das PCR-Verfahren (Polymerase Chain Reaction) gemäss Ausubel et al., Current Protocols in Molecular Biology, Green Publishing Associates Wiley Interscience, New York, (1989) amplifiziert. Für die PCR wurden folgende Primer verwendet:
*MADSA:* 
SaAEN: 5'-CCGAATTCCATGGTGAGGGAAAAACA-3'
AtAEN: 5'-CCGAATTCCATGGTGAGGGCAAAACT-3'
SaAEB: 5'-CCGAATTCGGATCCTCACTTTCTGGAAGAACA-3'
AtAEB: 5'-CCGAATTCGGATCCTCACTTTCTTGAAGAACA-3'
*MADSB:*

SaBEN: 5'-CCGAATTCCATGGGAAGGGGTAGGGTT-3'
AtBEN: 5'-CCGAATTCCATGGGAAGAGGTAGGGTT-3'
SaBEB: 5'-CCGAATTCGGATCCCTACTCGTTCGAAGTGGT-3'
AtBEB: 5'-CCGAATTCGGATCCCTACTCGTTCGTAGTGGT-3'

Neben der homologen Region enthielten die Primer AEN und BEN jeweils noch eine EcoRI und eine NcoI Schnittstelle und die Primer AEB und BEB eine EcoRI sowie eine BamHI Schnittstelle. Die amplifizierten Produkte wurden nach EcoRI-Verdau in die EcoRI-Schnittstelle des Vektors pBS SK⁺ (Stratagene) ligiert. Die Insertionen von selektionierten Klonen wurden sequenziert, um mögliche Fehler der PCR auszuschliessen. Die codierenden Bereiche wurden anschliessend mit NcoI und BamHI aus dem Vektor ausgeschnitten, über ein Agarosegel gereinigt und in den Vektor pSH9 (Holtorf et al. Plant Mol. Biol., 29, 637-646, 1995) inseriert. Dieser Vektor enthält den 35S Promotor und das Polyadenylierungssignal aus dem Cauliflower-Mosaic-Virus (CaMV). Diese sogenannte Expressionskassette wurde anschliessend mit HindIII ausgeschnitten und in den binären Vektor BIN19 (Bevan, Nucl. Acids Res. 12, 8711-8721, 1984) ligiert. Nach der Vermehrung der rekombinanten Plasmide in *E. coli* wurden diese in Agrobakterien transformiert (Höfgen, R. und Willmitzer, L., Nucl. Acids Res. 16, 9877, 1988). Agrobakterien, die die rekombinanten Plasmide enthielten, wurden zur Pflanzentransformation verwendet.

### 1.3. Transformation von Arabidopsis.

Die Transformation von *Arabidopsis* durch die Vakuum-Infiltration erfolgte analog zu dem beschriebenen Verfahren nach Bechthold et al., C. R. Acad. Sci. Paris, 316, 1194-1199, 1993.

### 1.4. Auswertung der transgenen Linien:

Von jedem Überexpressionskonstrukt (Sense) wurden jeweils 10 transgene Linien untersucht. Da die Länge der vegetativen Phase bei *Arabidopsis* mit der Anzahl der gebildeten Rosettenblätter korreliert, werden in der Regel die Anzahl der gebildeten Blätter bis zur Blütenbildung als Mass für den Blühzeitpunkt ausgewertet.

| **Genotyp** | **Blätter im Kurztag** | **Blätter im Langtag** |
|---|---|---|
| Col WT | 65,1 | 16,3 |
| *35S::SaMADSA*7 | 36,7 | 12,3 |
| *35S::SaMADSB*21 | 37,4 | 7,0 |
| *35S::AtMADSA*1 | 11,2 | 7,5 |
| *35S::AtMADSB*1 | 10,3 | 7,2 |
| *35S::AtFPF1* | 43,3 | 11,4 |
| *35S::SaLFY* | 46,6 | 14,2 |
| *35S::SaAP1* | 58,4 | 9,6 |

Von den 10 pro Konstrukt ausgewerteten transgenen Linien wurden die Werte der am frühesten blühenden Linie in die Tabelle aufgenommen. Eingetragen ist jeweils die Gesamtzahl der Blätter, inklusive der Hochblätter an der Hauptachse der Infloreszenz.

Aus der Tabelle ist zu ersehen, dass die transgenen Pflanzen in beiden Photoperioden deutlich weniger Blätter produzieren und damit auch früher blühen als die Kontrollpflanzen. Auffällig ist das besonders frühe Blühen der Linien, die die *Arabidopsis* cDNAs von *MADSA* und *MADSB* überexprimieren. Dies ergab sich durch eine bessere Transformationsausbeute, so dass im Gegensatz zur Transformation mit den *SaMADSA* und *SaMADSB* cDNAs bereits unter den Primärtransformanden nach Frühblühern selektioniert werden konnte.

### Beispiel II

### Kreuzung von Linien, die verschiedene Gene überexprimieren

### 2.1. Kreuzungsversuche

Es wurde Kreuzungen folgender Linien durchgeführt:
*35S::AtFPF1* X *35S::SaMADSA*
*35S::AtFPF1* X *35S::SaMADSB*
*35S::SaMADSA* X *35S::SaMADSB*
*35S::AtFPF1* X *35S::SaAP1*
*35S::ALFPF1* X *35S::SaLFY*

Für die Kreuzungen wurden die noch geschlossenen Blütenknospen der Empfängerpflanzen mit einer Pinzette geöffnet und die Pollensäcke der Blüten entfernt, um eine Selbstbestäubung zu vermeiden. Der Pollen, der oben als erstes aufgeführten Linien wurde dann jeweils auf die Narben der geöffneten Knospen übertragen. Nach 4 Wochen wurden reife Schoten geerntet und die Samen für die weiteren Untersuchungen ausgesät.

### 2.2. Auswertung der Kreuzungen

| **Genotyp** | **Blätter im Kurztag** | **Blätter im Langtag** |
|---|---|---|
| Col WT | 65,1 | 16,3 |
| *35S::AtFPF1* X *35S::SaMADSA* | 16,2 | 6,3 |
| *35S::AtFPF1* X *35S::SaMADSB* | 11,5 | 7,8 |
| *35S::SaMADSA* X *35S::SaMADSB* | 35,5 | 10,1 |
| *35S::AtFPF1* X *35S::SaAP1* | 9,8 | 8,6 |
| *35S::AtFPF1* X *35S::SaLFY* | 17,3 | 11,6 |

Von den Kreuzungen der transgenen Pflanzen wurden zunächst doppelt homozygote Linien selektioniert. Von den selektionierten Linien wurden jeweils 12 Pflanzen angezogen und ausgewertet.

In allen Linien, in denen *35S::AtFPF1* hineingekreuzt worden war, zeigte sich eine deutliche Reduktion der Zeitdauer bis zu Blütenbildung. Die Pflanzen die *MADSA* und *MADSB* überexprimierten, zeigten keine weitere Verkürzung der vegetativen Phase.

### Beispiel III

### Herstellung von Pflanzenlinien mit zwei Transgenen

### 3.1. Herstellung von Transformationsvektoren, die zwei Gene unter der Kontrolle von verschiedenen Promotoren beinhalten

Die codierenden Bereiche von Sa*MADSA*, *SaMADSB* und *AtFPF1* wurden wie unter Beispiel I beschrieben in den pSH5-Vektor, der einen Ubiquitin-Promotor enthält (Holtorf et al., supra), ligiert. Die klonierten Expressionkassetten wurden dann mit PstI ausgeschnitten und über ein Gel aufgereinigt und in den pBS SK⁺-Vektor ligiert. Aus dem pBS SK⁺-Vektor konnten die Fragmente mit dem Ubiquitin-Promotor, dem jeweiligen codierenden Bereich und dem CaMV-Terminator dann mit BamHI und EcoRI ausgeschnitten und in die entsprechenden *pBIN19-MADSA, pBIN19-MADSB* und pBIN19-*FPF1* Vektoren eingesetzt werden, in denen die codierenden Bereiche der entsprechenden Gene vom CaMV-Promotor kontrolliert werden. Erhalten wurden die Transformationsvektoren: pBIN19-*MADSA-MADSB*, pBIN19-*MADSA-FPF1* und pBIN19-*MADSB-FPF1*. Diese Vektoren wurden anschliessend in E. coli vermehrt und in Agrobakterien transformiert. *Arabidopsis* Pflanzen wurden mit der Infiltrationsmethode nach Bechthold et al. (supra) transformiert.

### 3.2. Analyse der transgenen Pflanzen

| **Genotyp** | **Blätter im Kurztag** | **Blätter im Langtag** |
|---|---|---|
| Col WT | 65,1 | 16,3 |
| *35S::AtMADSA-UBI::AtMADSB* | 38,3 | 12,1 |
| *35S::AtMADSA-UBI::AtFPF1* | 18,2 | 8,4 |
| *35S::AtMADSB-UBI::AtFPF1* | 19, 6 | 7,8 |

Auch in diesem Versuch zeigte sich, dass Pflanzen mit zwei Transgenen deutlich früher blühen als die Kontrollpflanzen. Eine Selektion von verschiedenen Blühzeitpunkten aus einer Vielzahl von unabhängigen Transformanden war ausserdem möglich.

### Beispiel IV

### Herstellung von Transformationsvektoren mit Fusionsproteinen zwischen FPF1 und MADSA bzw FPF1 und MADSB

### 4.1. Herstellung der Konstrukte und Transformation von Pflanzen

In die rekombinanten Vektoren pBIN19-MADSA, pBIN19-MADSB und pBIN19-*FPF1* wurden PCR-Fragmente der codierenden Bereiche von *MADSA, MADSB* und *FPF1*, die jeweils eine NcoI-Schnittstelle am Startcodon und nach der letzten codierten Aminosäure aufwiesen, in die NcoI Schnittstelle eingefügt. Dadurch wurden rekombinante Vektoren erzeugt, die zwei codierende Regionen unter der Kontrolle des CaMV-Promotors enthielten. Erhalten wurden die vier Konstrukte 35S::*MADSA*::*FPF1*, 35S::*MADSB*::*FPF1*, 35S::*FPF1*::*MADSA* und 35S::*FPF1*::*MADSB*. Die rekombinanten Vektoren wurden in E. coli vermehrt und in Agrobakterien überführt. Arabidopsis wurde nach Bechthold et al. (supra) transformiert.

### 4.2. Analyse der transgenen Pflanzen.

Die transgenen Pflanzen blühten deutlich früher als entsprechende Kontrollpflanzen und als Pflanzen, die jeweils nur ein Gen überexprimieren. Ausgewertet wurden je 8 transformierte Linien mit jeweils 10 Pflanzen. Die Werte der frühesten Linien sind in der Tabelle dargestellt.

| **Genotyp** | **Blätter im Kurztag** | **Blätter im Langtag** |
|---|---|---|
| Col WT | 65, 1 | 16,3 |
| 35S::*AtMADSA*::*AtFPF1* | 21,3 | 11,2 |
| 35S::*AtMADSB*::*AtFPF1* | 18,2 | 10,8 |
| 35S::*AtFPF1*::*AtMADSA* | 23,8 | 12,1 |
| 35S::*AtFPF1*::*AtMADSB* | 22,7 | 12,3 |

In diesem Versuch zeigte sich, dass Pflanzen mit zwei Transgenen unter der Kontrolle von nur einem Promotor auch deutlich früher blühen als die Kontrollpflanzen. Eine Selektion von verschiedenen Blühzeitpunkten aus einer Vielzahl von unabhängigen Transformanden war hier ebenso möglich.

### Beispiel V

### Veränderung des Blühzeitpunktes in transgenen Tabakvarietäten mit unterschiedlichen photoperiodischen Abhängigkeiten zur Blühinduktion

Seit der Endeckung der photoperiodischen Induktion der Blütenbildung (Garner und Allard, J. Agric. Res. 18, 553-606, 1920) sind unzählige Studien durchgeführt worden, um den Einfluss der Tageslänge auf die Blühinduktion zu verstehen. Die meisten der Pflanzenarten die hierzu verwendet wurden, zeigen eine strikte Abhängigkeit von der Photoperiode, d.h. sie blühen nur, wenn eine kritische Dauer der Lichtperiode unterschritten (Kurztagpflanzen) bzw. überschritten wird (Langtagpflanzen). Zu diesen Pflanzen gehörten insbesondere auch die verschiedenen Tabakarten mit verschiedenen photoperiodischen Ansprüchen für eine Blühinduktion. In den hier beschriebenen Beispielen wurden drei verschiedene Tabakarten verwendet: der Langtag-Tabak *Nicotiana sylvestris* (Ns), der tagneutrale Tabak *Nicotiana tabacum* (Nt) und der Kurztag-Tabak *Nicotiana tabacum* Maryland Mammoth (Nt-MM). Durch die Verwendung der in den vorangehenden Beispielen vorgestellten Genkonstrukte kann eine Blühinduktion der strikt photoperiodischen Tabakvarietäten auch unter nichtinduzierenden Photoperioden erfolgen.

### 5.1. Transformation von Tabak

Zur Transformation der verschiedenen photoperiodischen Tabakvarietäten wurden die Konstrukte, die im Beispiel I beschrieben wurden verwendet. Zusätzlich wurde auch noch das homologe *FPF1* Gen aus *Nicotiana tabacum* eingesetzt, das eine Identität der Nukleotidsequenz in der codierenden Region von 67,7% zu dem *FPF1* Gen aus dem Senf aufweist. Dieses Tabak *FPF*-Gen wurde in gleicher Weise für eine konstitutive Expression mit einem CaMV Promotor und einem Terminator versehen, wie es im Beispiel 1 für die Senf- und Arabidopsis-Transgene beschrieben worden ist. Hierzu wurden durch eine PCR-Reaktion mit folgenden Primern je eine *NcoI* Restriktionsschnittstelle am Startcodon und eine *Bam*HI. Restriktionsschnittstelle am Stopcodon eingeführt:

NtFPF-EN: 5' CAGGAATTCCATGGCTGGAGTTTGGGT 3'
NtFPF-EB: 5' CAGGAATTCGGATCCTTATCATATGTCTCTAAC 3'

Die Transformation von Tabak wurde mit einer Stanadardmethode durchgeführt (Horsch et al. Science 227, 12229-1234, 1985).

### 5.2. Konstitutive Expression von FPF1, MADSA oder MADSB

Die transgenen Pflanzen wurden unter den gleichen Kurz- bzw. Langtagbedingungen in Klimmakammern angezogen, wie sie auch für *Arabidopsis* verwendet wurden.

Für die Auswertung des Blühzeitpunktes wurde im Falle des Tabaks die Zeitdauer von der Aussaat bis zum Oeffnen der 1. Blüte berücksichtigt. Ausgewertet wurden von einer repräsentativen Linie jeweils 8 Pflanzen. In der folgenden Tabelle sind Pflanzen berücksichtigt, die jeweils nur ein Gen konstitutiv überexprimieren.

| **Genotyp** | **Anzahl der Ta- ge bis zur Blütenbildung im KT** | **Anzahl der Ta- ge bis zur Blütenbildung im LT** |
|---|---|---|
| Nt | 93 | 76 |
| Nt 35S::*SaFPF1* | 85 | 68 |
| Nt 35S::*SaMADSA* | 76 | 55 |
| Nt 35S::*SaMADSB* | 68 | 54 |
| Nt 35S::*NtFPF1* | 81 | 64 |
| Nt-MM | 106 | nicht blühend |
| Nt-MM 35S::*SaFPF1* | 99 | nicht blühend |
| Nt-MM 35S::*SaMADSA* | 72 | 124 |
| Nt-MM 35S::*SaMADSB* | 80 | nicht blühend |
| Nt-MM 35S::*NtFPF1* | 97 | nicht blühend |
| Ns | nicht blühend | 82 |
| Ns 35S::*FPF1* | nicht blühend | 78 |
| Ns 35S::*MADSA* | nicht blühend | 76 |
| Ns 35S::*MADSB* | 94 | 70 |
| Ns 35S::*NtFPF1* | nicht blühend | 67 |

Die Auswertung dieses Versuches zeigt, dass der tagneutrale Tabak *Nicotiana tabacum* durch die Überexpression der verschiedenen Transgene sowohl unter Kurztag- als auch unter Langtagbedingungen schneller zum Blühen kommt. Der Blüten- und Samenansatz ist in allen Fällen vergleichbar mit dem Ansatz in Wildtyppflanzen. Der transgene Kurztagtabak *Nicotiana tabacum* Maryland Mammoth blüht unter induzierenden Kurztagbedingungen jeweils früher als die Wildtyp-Pflanzen unter den gleichen Bedingungen. Unter nichtinduzierenden Langtagbedingungen blüht der Wildtyp Maryland Mammoth Tabak nicht. Transgener Maryland Mammoth Tabak der *FPF1* oder *MADSB* überexprimiert blüht auch nicht unter Langtagbedingungen, wird jedoch MADSA überexprimiert, dann blüht auch dieser Tabak unter nichtinduzierenden Bedingungen. Durch die Überexpression nur eines einzelnen Genes wird hier also die photoperiodische Schranke der Blühinduktion unter nichtinduzierenden Bedingungen überwunden. *Nicotiana sylvestris* Wildtyppflanzen blühen nicht unter Kurztagbedingungen und auch die konstitutive Expression von *FPF1* oder *MADSA* führt nicht zur Blüte unter nichtinduzierenden Bedingungen. Die Überexpression von *MADSB* führt jedoch auch im Langtagtabak *Nicotiana sylvestris* zu einer Blütenbildung unter nichtinduzierenden Kurztagbedingungen.

### Beispiel VI

### 6.1. Kombinierte Expression von FPF1 mit MADSA oder MADSB in verschiedenen Tabakvarietäten

Analog zu den im Beispiel II beschriebenen Kombinationen vor. Transgenen durch Kreuzungen, wurden auch mit den verschiedenen photoperiodischen Tabaklinien, die *FPF1*, *MADSA* oder *MADSB* überexprimieren, Kreuzungen durchgeführt. In der folgenden Tabelle sind die Blühzeitpunkte von Pflanzen die jeweils zwei Transgene enthalten aufgeführt.

| **Genotyp** | **Anzahl der Tage bis zur Blütenbildung im KT** | **Anzahl der Tage bis zur Blütenbildung im LT** |
|---|---|---|
| Nt 35S::*SaFPF1* X Nt 35S::*SaMADSA* | 65 | 59 |
| Nt 35S::*FPF1* X Nt 35S::*SaMADSB* | 60 | 50 |
| Nt-MM 35S::*SaFPF1* X Nt-MM 35S::*SaMADSA* | 68 | 88 |
| Nt-MM 35S::*SaFPF1* X Nt-MM 35S::*SaMADSB* | 76 | 98 |
| Ns 35S::*SaFPF1* X Ns 35S::*SaMADSA* | nicht blühend | 67 |
| Ns 35S::*SaFPF1* X Ns 35S::*SaMADSB* | 82 | 62 |

Bis auf die Kreuzung *Ns-SaFPF1* mit *Ns-SaMADSA* führt die kombinierte Expression in allen Fällen dazu, dass die vegetativen Phasen weiter verkürzt werden. Während Maryland Mammoth Pflanzen die entweder *MADSB* oder *FPF1* überexprimieren, keine Blüten unter nichtinduzierenden Langtagbedingungen ansetzten, führt die kombinierte Expression dieser beiden Gene unter sonst gleicher. Bedingungen jedoch zur Blütenbildung.

### Beispiel VII

### Modifizierung des Blühzeitpunktes in Rapspflanzen

Raps ist eine agronomisch wichtige Pflanze, die auf allen Kontinenten zur Herstellung von Speise- und Industrieölen angebaut wird. In nördlichen Breitengraden wie z.B. in Kanada oder Skandinavien besteht in den Rapsanbauregionen die Gefahr von frühen Wintereinbrüchen, die häufig die Rapsernte entwerten, da der Raps dann nicht ausreifen kann und nur minderwertiges Öl liefert. Eine Verfrühung des Blühzeitpunktes und damit eine frühere Reife der Rapspflanzen um wenige Tage könnte dieses Problem lösen. Weiterhin könnten frühblühende Rapspflanzen noch weiter nördlich angebaut und damit die Anbaufläche ausgedehnt werden.

### 7.1. Herstellung transgener Rapspflanzen

Für eine Überexpression in Rapspflanzen *(Brassica napus)* wurden die in Beispiel I beschriebenen Vektoren zur Expression von *FPF1, MADSA* und *MADSB* verwendet. Die Transformation erfolgte nach einer Standardmethode (Moloney et al., Plant Cell Reports, 8, 238-242, 1989). Transformiert wurden eine Winter- (WR) und eine Sommerrapslinie (SR).

### 7.2. Analyse des Blühzeitpunktes der transgen Rapspflanzen

Aufgenommen in die Tabelle wurden die Anzahl der Tage, die der Raps früher reif war als entsprechende Kontrollpflanzen. Ausgewertet wurden jeweils 12 Pflanzen von einer repräsentativen transgenen Linie. Die Pflanzen wurden in Gewächshäusern angezogen und wie beim Winterraps notwendig für verschiedene Zeiten Vernalisationsbedingungen ausgesetzt.

| **Genotyp** | **Anzahl der Tage, die der transgene Raps früher reif war** |
|---|---|
| Bn (WR) 35S::*SaFPF1* | 7 |
| Bn (SR) 35S::*SaFPF1* | 3 |
| Bn (WR) 35S::*SaMADSA* | 7 |
| Bn (SR) 35S::*SaMADSA* | 5 |
| Bn (WR) 35S::*SaMADSB* | 12 |
| Bn (SR) 35S::*SaMADSB* | 6 |

Die transgenen Rapspflanzen wurden signifikant früher reif als die Wildtyppflanzen unter gleichen Bedingungen. Es zeigte sich ferner, dass Winterrapspflanzen, die das *MADSB* Gen überexprimieren, deutlich kürzer vernalisiert werden müssen, um zur Blütenbildung zu gelangen. Während Wildtyppflanzen für 8 Wochen bei 4°C gehalten werden müssen, waren für 35S::*MADSB* Pflanzen nur 2 Wochen Vernalisation notwendig für eine vollständige Kompetenz zur Blühinduktion. *Die Kombination von 355::MADSB mit 35S::*FPF1 führte hierbei sogar zur einer vollständigen Aufhebung der Vernalisationsnotwendigkeit für die Blütenbildung. Dies kann zur schnelleren Züchtung von Wintergetreiden und Winterrapspflanzen bzw. zur Aussaat der Samen nach der Winterperiode ausgenützt werden.

### Beispiel VIII

### Herstellung von Transformationsvektoren mit Antisensekonstrukten zur Verhinderung der Blütenbildung

### 8.1. Herstellung von Transformationsvektoren mit AntisenseKonstrukten von FPF1, MADSA und MADSB

Die Antisense-Konstrukte finden Anwendung z. B. beim Anbau von Zuckerrüben und Salatpflanzen. Das Verfahren wird hier modellhaft an *Arabidopsis thaliana* durchgeführt.

### Antisense-Konstrukte:

Durch die Transformation von Pflanzen mit DNA-Konstrukten, die die Transkription einer Antisense-RNA in der Pflanze ermöglichen, kann die Expression eines Genes so unterdrückt werden, dass aus den eventuell auftretenden phänotypischen Veränderungen der transformierten Pflanze auf die Funktion dieses Genes in Stoffwechsel- oder Entwicklungsprozessen geschlossen werden kann. Um eine spezifische Inhibition der Expression von *AthMADSA* und *AthMADSB* ohne eine gleichzeitige Beeinflussung der Aktivität anderer MADS Box Gene zu erzielen, wurden für die Herstellung der Transformationskonstrukte diejenigen Abschnitte der Arabi*dopsis* cDNAs verwendet, die den konservierten MADS Box Bereich nicht enthielten. In einem ersten Schritt wurde ein 530 bp umfassendes XbaI/HindIII-Fragment der *AthMADSA* cDNA, das einen Teil des codierenden und den fast vollständigen 3' nicht-codierenden Bereich enthält, sowie ein 640 Basenpaare (bp) langes BamHI/HindIII-Fragment der *AthMADSB* cDNA, das ebenfalls einen Teil des codierenden Bereiches sowie den vollständigen 3 nicht-codierenden Abschnitt beinhaltet, ausgeschnitten. Die überstehenden Enden der isolierten Fragmente wurden aufgefüllt und in die SmaI-Schnittstelle des pBS SK⁺-Vektors (Stratagene) ligiert.

Für die Herstellung des *FPF1* Antisensekonstruktes konnte die volständige cDNA mit BamHI und EcoRI in der richtigen Orientierung aus einem pBS SK⁺-Vektor herausgeschnitten werden.

Nach Bestimmung der geeigneten Orientierung der *MADSA* und *MADSB* cDNAs konnten alle drei cDNAs mit BamHI und EcoRI isoliert und gerichtet in Antisense-Orientierung in den Vektor pRT 104 'Töpfer et al., Nucl. Acids Res. 15, 5890, 1987) ligiert werden. Dadurch entstand eine Promotor::Antisense::Terminator-Kassette, bestehend aus dem CaMV 35S Promotor, der jeweiligen cDNA (*MADSA*, *MADSB* oder *FPF1*) und einem CaMV-Polyadenylierungssignal. Zur Überprüfung der Antisense-Orientierung der cDNA-Fragmente wurden die Konstrukte sequenziert.

Die Antisense-Konstrukte wurden dann durch einen HindIII-Verdau aus dem Vektor pRT104 isoliert und in die HindIII-Schnittstelle des Pflanzentransformationsvektors pBIN19 (Bevan, supra) inseriert. Die einzelnen Schritte der Klonierung wurden durch Southern Blot-Analysen verfolgt.

Die rekombinanten BIN19-Plasmide wurden in E. coli kloniert und anschliessend in Agrobakterien überführt. *Arabidopsis* wurde nach Bechthold et al., (supra) transformiert.

### 8.2. Analyse der transgenen Pflanzen

| **Genotyp** | **Blätter im Kurztag** | **Blätter im Langtag** |
|---|---|---|
| Col WT | 65,1 | 16,3 |
| *35S::ASAtFPF1* | 79,8 | 18,2 |
| *35S::ASAtMADSA*40 | 73,3 | 21,0 |
| *35S::ASAtMADSB*74 | 76,5 | 17,9 |
| *35S::ASAtMADSA*40 X *35S::ASAtMADSB*74 | 86,3 | 25,8 |

Es konnte somit gezeigt werden, dass transgene Linien mit Antisensekonstrukten deulich später blühen als entsprechende Kontrollpflanzen.

## Patentansprüche

1. Rekombinante DNA-Sequenz, die wenigstens drei DNA-Sequenzen umfasst, **dadurch gekennzeichnet, dass** die erste DNA-Sequenz eine regulatorische Sequenz umfasst, welche die Expression eines DNA-Fragments in einem Organismus steuert und die zweite und dritte DNA-Sequenz codierende Sequenzen zweier unterschiedlicher Blühinduktionsgene umfassen, selektiert von der Gruppe bestehend aus MADSA, MADSB, FPF1 sowie codierende Sequenzen homologer Gene mit einer Identität von 60 bis 98 %.

2. Rekombinante DNA-Sequenz gemäss Anspruch 1, **dadurch gekennzeichnet, dass** die codierenden Sequenzen die Gene MADSA und FPF1 oder MADSB und FPF1 umfassen.

3. Rekombinante DNA-Sequenz gemäss Ansprüchen 1 oder 2, **dadurch gekennzeichnet, dass** sie die Vorverlegung der Blühinduktion in Nutz- und Zierpflanzen bewirken.

4. Verfahren zur Vorverlegung der Blühinduktion in Nutz- und Zierpflanzen, **dadurch gekennzeichnet, dass** die codierenden Bereiche zweier unterschiedlicher Blühinduktionsgene selektiert von der Gruppe bestehend aus MADSA, MADSB und FPF1 konstitutiv exprimiert werden, jeweils unter Kontrolle einer eigenen regulatorischen Sequenz stehen, und dass die codierenden Bereiche der besagten Gene MADSA, MADSB und FPF1 unter Kontrolle eines CaMV-Promotors, oder unter Kontrolle von anderen Promotoren mit Expressionssignalsequenzen, einschliesslich der bakteriellen Promotoren des Nopalin-Synthetase-Gens (nos), des Octopin-Synthetase-Gens (ocs) des Ti-Plasmids von *Agrobacterium tumefaciens*, oder der Ubiquitin-, Actin-, Histon- und Tubulin-Promotoren, oder der Hitzeschock- und Abscissinsäure (ABA)-induzierbaren Promotoren oder von meristemspezifischen Promotoren, stehen.

5. Verfahren nach Anspruch 4, **dadurch gekennzeichnet, dass** die codierenden Bereiche der Gene MADSA, MADSB und FPF1 in einen Vektor eingebracht werden.

6. Verfahren nach einem der Ansprüche 4 oder 5, **dadurch gekennzeichnet, dass** die codierenden Bereiche der Gene MADSA, MADSB und FPF1
a) in einen einen Ubiquitin-Promotor und einen CaMV-Terminator enthaltenden pSH5-Vektor ligiert werden;
b) die aus a) resultierenden Expressionskassetten in CaMV-Promotoren enthaltende pBIN19-MSDSA, pBIN19-MASDSB und pBIN19-FPF1-Vektoren ligiert werden;
c) die aus b) resultierenden Konstrukte 3SS::MADSA::UBI::FPF1, 35S::MADSB::UBI::FPF1, 35S::FPF1::UBI::MADSA und 35S::FPF1::UBI::MADSB in E. coli vermehrt werden; und
d) die aus c) erhaltenen Konstrukte in Pflanzen, vorzugsweise Nutz- und Zierpflanzen, transformiert werden.

7. Verfahren nach Anspruch 6, **dadurch gekennzeichnet, dass** die in Schritt d) genannten Nutzpflanzen Pflanzen aus den Gattungen Triticum, Oryza, Zea, Hordeum, Sorghum, Avena, Secale, Lolium, Festuca, Lotus, Medicago, Glycine, Brassica, Solanum, Beta, sowie Gemüse oder Früchte produzierende Pflanzen, angiosperme Bäume und Zierpflanzen umfassen.

8. Rekombinante DNA-Sequenz gemäss Anspruch 1 bis 3, **dadurch gekennzeichnet, dass** die wirksamen Domänen der Blühinduktionsgene als Fusionsprotein exprimiert werden.

9. Verfahren gemäss Anspruch 4, **dadurch gekennzeichnet, dass** die wirksamen Domänen der Blühinduktionsgene als Fusionspmtein exprimiert werden.

10. Verfahren gemäss Anspruch 9 **dadurch gekennzeichnet, dass**
a) in die rekombinanten Vektoren pBIN19-MADSA, pBIN19-MADSB und pBIN19-FPF1 Fragmente der codierenden Bereiche von MADSA, MADSB und FPF1 eingefugt werden;
b) die aus a) erhaltenen Konstrukte 35S::MADSA::FPF1, 35S: :MADSB:: FPF1, 35S::FPF1::MADSA und 35S::FPF1::MADSB in E. coli vermehrt werden; und
c) die in b) vermehrten Konstrukte in Pflanzen, vorzugsweise in Nutz- und Zierpflanzen, transformiert werden.

11. Verfahren nach Anspruch 10, **dadurch gekennzeichnet, dass** die in Schritt c) genannten Nutzpflanzen Pflanzen aus den Gattungen Triticum, Oryza, Zea, Hordeum, Sorghum, Avena, Secale, Lolium, Festuca, Lotus, Medicago, Glycine, Brassica, Solanum, Beta, sowie Gemüse oder Früchte produzierende Pflanzen, angiosperme Bäume und Zierpflanzen umfassen.

12. Verfahren zur Verzögerung der Blühinduktion in Nutz- und Zierpflanzen, **dadurch gekennzeichnet, dass** die codierenden Sequenzen zweier unterschiedlicher Blühinduktionsgene in Antisense-Orientierung in einer Pflanze exprimiert werden, wobei die Antisense Konstrukte aus den Genen FPF1, MADSA oder MADSB hergeleitet werden.

13. Verfahren nach Anspruch 12, **dadurch gekennzeichnet, dass** zwei Elternpflanzenflanzen, die jeweils eine codierende Sequenz von zwei unterschiedlichen Blühinduktionsgene in Antisense-Orientierung exprimieren, miteinander gekreuzt werden, wobei die Antisense Konstrukte aus den Genen FPF1, MADSA oder MADSB hergeleitet werden..

14. Verfahren nach Anspruch 13, **dadurch gekennzeichnet dass** die Expression der codierenden Sequenzen der beiden verschiedenen Blühinduktionsgene in Antisense-Orientierung in den beiden Elternpflanzen zu keiner nennenswerten Reduktion der Blütenbildung führen.

15. Verfahren nach den Ansprüchen 13 und 14, **dadurch gekennzeichnet, dass** es sich bei den beiden Elternpflanzen um Zuckerrüben handelt.

16. Verfahren nach Anspruch 12, **dadurch gekennzeichnet, dass** eine rekombinante DNA-Sequenz gemäss den Ansprüchen 1 oder 2 in Antisense-Orientierung exprimiert wird.

17. Verfahren zur Verkürzung der Vernalisationszeit von Wintergetreiden und Winterraps, **dadurch gekennzeichnet, dass** eine rekombinante DNA-Sequenz gemäss einem der Ansprüche 1 oder 2 zur Herstellung von transgenem Winterraps und Wintergetreide verwendet wird.

18. Verfahren nach Anspruch 17, **dadurch gekennzeichnet, dass** die genannte rekombinante DNA-Sequenz eine Überexpression des MADSB-Gens bewirkt.

19. Verfahren zur Aufhebung der Vemalisationszeit von Winterraps und Wintergetreiden, **dadurch gekennzeichnet, dass** eine rekombinante DNA-Sequenz gemäss Anspruch 17 zur Herstellung von transgenem Winterraps und transgenen Wintergetreiden verwendet wird, die eine kombinierte Überexpression der MADSB- und FPF1-Gene bewirkt.

## Claims

1. A recombinant DNA-sequence, which comprises at least three DNA-sequences, **characterised in that**, that the first DNA-sequence comprises a regulatory sequence, which affects the expression of a DNA-fragment in an organism, and the second and third DNA-sequence encoded sequences comprise two different bloom-induction genes, selected from the group consisting of MADSA, MADSB, FPFI as well as encoded sequences of homologous genes with an identity of 60 to 98%.

2. A recombinant DNA-sequence according to Claim 1, **characterised in that**, that the encoded sequences comprise the genes MADSA and FPFI or MADSB and FPFI.

3. A recombinant DNA-sequence according to Claims 1 or 2, **characterised in that**, that they bring about an earlier date of bloom- induction in food and ornamental plants.

4. A procedure for bringing about bloom-induction at an earlier date in food and ornamental plants, **characterised in that**, that the encoded fields of two different bloom inducing genes selected from the group consisting of MADSA, MADSB and FPFI are expressed constitutively, respectively standing under control of an inherent regulating sequence, and that the encoded field of the said genes MADSA, MADSB and FPFI are under control of a CaMV- promoter, or under control of other promoters with expression-signal-sequences, inclusive of the bacterial promoters of the Nopalin-synthetase-gene (nos), the Octopin-synthetase-gene (ocs), the Ti-plasmids of *Agrobacterium tumefaciens,* or the Ubiquitin-, Actin-, Histon- and Tubulin-promoters, or the heat shock- and abscissa acid (ABA)-inducing promoters, or from meristem-specific promoters.

5. A procedure according to Claim 4, **characterised in that**, that the encoded fields of the genes MADSA, MADSB and FPFI are introduced into a vector.

6. A procedure according to one of Claims 4 or 5, **characterised in that**, that the fields of genes MADSA, MADSB and FPFI
a) are ligated in an Ubiquitin-promoter and a CaMV-terminator containing a pSH5-vector;
b) the expression cassettes resulting from a) are ligated in CaMV-promoters containing pBIN19-MSDSA, pBIN19-MASDSB and pBIN19-FPFI - vectors ;
c) the constructs resulting from b) 3SS::MADSA::UBI::FPFI, 35SS::MADSB::UBI::FPFI, 35S::FPFI::UBI::MADSA and 35S::FPGI::UBI::MADSB are proliferated in E.coli ; and
d) the constructs in plants obtained from c) are transformed , preferably into food- and ornamental plants.

7. A procedure according to Claim 6, **characterised in that**, that the food plants named in step d) comprise the generic Triticum, Oryza, Zea, Hordeum, Sorghum, Avena, Secale, Lolium, Festuca, Lotus, Medicago, Glycine, Brassica, Solanum, Beta as well as vegetables or fruit producing plants, angiosperme trees and ornamental plants.

8. A recombinant DNA-sequence according to Claims 1 to 3, **characterised in that**, that the active domains of the bloom-inducing genes are expressed as a fusion protein.

9. A procedure according to Claim 4, **characterised in that**, that the active domains of the bloom inducing genes are expressed as a fusion protein.

10. A procedure according to Claim 9, **characterised in that**, that
a) in the recombinant vectors pBIN19-MADSA, pBIN-MADSB and pBIN19-FPFI, fragments of the encoded range of MADSA, MADSB and FPFI were inserted
b) the constructs obtained from a) 35S::MADSA::FPFI, 35S::MADSB::FPFI, 35S::FPFI::MADSA and 35S::FPFI::MADSB are proliferated in E.coli; and
c) the constructs proliferated in plants in b), are transformed preferably into food- and ornamental plants.

11. A procedure according to Claim 10, **characterised in that**, that the food plants named in step c) comprise the generic Triticum, Oryza, Zea, Hordeum, Sorghum, Avena, Secale, Lolium, Festuca, Lotus, Medicago, Glycine, Brassica, Solanum, Beta as well as vegetables or fruit producing plants, angiosperme trees and ornamental plants.

12. A procedure for retarding the bloom induction in food- and ornamental plants, **characterised in that**, that the encoded sequences of two different bloom-inducing genes are expressed in an antisense-orientation in a plant, whereby the antisense constructs are derived from the genes FPFI, MADSA or MADSB.

13. A procedure according to Claim 12, **characterised in that**, that two parental- plant plants which respectively express an encoded sequence of two different bloom-inducing genes in an antisense-orientation, are crossed with each other, whereby the antisense constructs are derived from the genes FPFI, MADSA or MADSB.

14. A procedure according to Claim 13, **characterised in that**, that the expression of the encoded sequences of both different bloom-inducing genes, in an antisense-orientation in both the parent plants, lead to no reduction of bloom formation worth mentioning.

15. A procedure according to Claims 13 and 14, **characterised in that**, that it relates to both parent plants of sugar beets.

16. A procedure according to Claim 12, **characterised in that**, that a recombinant DNA-sequence is expressed according to Claims 1 or 2 in an antisense-orientation.

17. A procedure for shortening the vernalisation time of winter grains and winter rapeseed, **characterised in that**, that a recombinant DNA-sequence is employed according to one of Claims 1 or 2 for manufacturing transgenic winter rapeseed and winter grains.

18. A procedure according to Claim 17, **characterised in that**, that the named recombinant DNA-sequence brings about an over-expression of the MADSB-gene.

19. A procedure for cancellation of the vernalisation time of winter rapeseed and winter grains, **characterised in that**, that a recombinant DNA-sequence is used according to Claim 17 for manufacturing transgenic winter rapeseed and transgenic winter grains, which brings about a combined over-expression of the MADSB- and FPFI-genes.

## Revendications

1. Séquence d'ADN recombinante, qui comprend au moins trois séquences d'ADN, **caractérisée en ce que** la première séquence d'ADN comprend une séquence régulatrice, qui contrôle l'expression d'un fragment d'ADN dans un organisme et qui comprend les séquences codantes des deuxième et troisième séquences d'ADN de deux gènes d'induction de floraison différents choisis dans le groupe constitué par MADSA, MADSB, FPF1 ainsi que des séquences codantes de gènes homologues avec une identité de 60 à 98 %.

2. Séquence d'ADN recombinante selon la revendication 1,
**caractérisée en ce que** les séquences codantes comprennent les gènes MADSA et FPFI ou MADSB et FPF1.

3. Séquence d'ADN recombinante selon les revendications 1 ou 2,
**caractérisé en ce qu'**elles provoquent l'avancement de l'induction de floraison chez les plantes cultivées et les plantes d'omement.

4. Procédé pour avancer l'induction de floraison chez les plantes cultivées et les plantes d'omement, **caractérisé en ce que** les domaines codants de deux gènes d'induction de floraison différents choisis dans le groupe constitué par MADSA, MADSB, FPF1 sont exprimés de manière constitutive, chacun étant sous contrôle d'une séquence régulatrice particulière, et **en ce que** les domaines codants desdits gènes MADSA, MADSB, FPFI sont sous contrôle d'un promoteur CaMV, ou sous contrôle d'autres promoteurs avec des séquences de signal d'expression, y compris des promoteurs bactériens du gène Nopaline-synthétase (nos), du gène octopin-synthétase (ocs) de Ti-plasmide de *Agrobacterium tumefaciens,* ou des promoteurs d'ubiquitine, d'actine, d'histone et de tubuline, ou des promoteurs susceptibles d'être induits par un choc de chaleur et l'acide ascissique (ABA) ou comme promoteurs spécifiques au méristème.

5. Procédé selon la revendication 4, **caractérisé en ce que** les domaines codants des gènes MADSA, MADSB et FPF1 sont introduits dans un vecteur.

6. Procédé selon l'une des revendications 4 ou 5, **caractérisé en ce que** les domaines codants des gènes MADSA, MADSB et FPF1
a) sont liés dans un vecteur pSHS contenant un promoteur d'ubiquitine ou un terminateur CaMV ;
b) sont liés aux vecteurs pBIN19-MSDA, pBIN19-MASDSB et pBIN19-FPFI contenant les cassettes d'expression résultant de
a) dans les promoteurs CaMV ;
c) sont multipliés dans E. coli à partir des constructions 35S::MADSA::UBI::FPF1, 35S::MADSB::UBI::FPF1, 35S::FPF1 ::UBI::MADSA et 35S::FPF1::UBI::MADSB résultant de b) ;
d) sont transformés dans des plantes, de préférence des plantes cultivées et des plantes d'omement à partir des constructions obtenues en c).

7. Procédé selon la revendication 6, **caractérisé en ce que** les plantes cultivées citées dans l'étape d) comprennent des plantes des types Tricitum, Oryza, Zea, Hordeum, Sorghum, Avena, Secale, Lolium, Festuca, Lotus, Medicago, Glycine, Brassica, Solanum, Béta, ainsi que des plantes produisant des légumes et des fruits, des arbres angiospermes et des plantes d'omement.

8. Séquence d'ADN recombinante selon les revendications 1 à 3,
**caractérisée en ce que** les domaines efficaces des gènes d'induction de la floraison sont exprimés comme protéine de fusion.

9. Procédé selon la revendication 4, **caractérisé en** de que les domaines efficaces des gènes d'induction de la floraison sont exprimés comme protéine de fusion.

10. Procédé selon la revendication 9, **caractérisé en ce que**
a) dans les vecteurs recombinants pBIN19-MADSA, pBIN19-MADSB et pBIN19-FPF1 sont joints des fragments des domines codants de MADSA, MADSB et FPFI ;
b) les constructions obtenues dans a) 35S::MADSA::FPF1, 35S::MADSB::FPF1, 35S::FPF1::MADSA et 35S::FPF1::MADSB sont multipliés dans E. coli ; et
c) les constructions multipliées en b) sont transformées dans des plantes, de préférence des plantes cultivées et des plantes d'ornement.

11. Procédé selon la revendication 10, **caractérisé en que** lesdites plantes cultivées citées dans l'étape d) comprennent des plantes des types Tricitum, Oryza, Zea, Hordeum, Sorghum, Avena, Secale, Lolium, Festuca, Lotus, Medicago, Glycine, Brassica, Solanum, Béta, ainsi que des plantes produisant des légumes et des fruits, des arbres angiospermes et des plantes d'omement.

12. Procédé de retardement de l'induction de floraison dans les plantes cultivées et les plantes d'omement, **caractérisé en ce que** les séquences codantes de deux gènes d'induction de la floraison différents sont exprimés dans une plante dans l'orientation anti-sens, les constructions anti-sens dérivant des gènes FPF1, MADSA ou MADSB.

13. Procédé selon la revendication 12, **caractérisé en ce qu'**on croise ensemble deux plantes parents qui chacune exprime une séquence codante de deux gènes d'induction de floraison différents dans une orientation anti-sens, les constructions anti-sens dérivant des gènes FPF1, MADSA ou MADSB.

14. Procédé selon la revendication 13, **caractérisé en ce** l'expression des séquences codantes des deux gènes d'induction de la floraison différents dans l'orientation anti-sens dans les deux plantes parents ne conduit pas notablement à la réduction de la formation de la floraison.

15. Procédé selon les revendications 13 et 14, **caractérisé en ce qu'**il s'agit de deux plantes parents de betteraves à sucre.

16. Procédé selon la revendication 12, **caractérisé en ce qu'**une séquence d'ADN recombinante selon les revendications 1 ou 2 est exprimée dans l'orientation anti-sens.

17. Procédé de raccourcissement de la durée de vemalisation de céréale d'hiver et de colza d'hiver, **caractérisé en ce qu'**on utilise une séquence d'ADN recombinante selon l'une des revendications 1 ou 2 pour la préparation de colza d'hiver ou de céréale d'hiver transgéniques.

18. Procédé selon la revendication 17, **caractérisé en ce que** ladite séquence d'ADN recombinante provoque une surexpression du gène MADSB.

19. Procédé pour la suppression de la durée de vernalisation de colza d'hiver et de céréale d'hiver, **caractérisé en ce qu'**on utilise une séquence d'ADN recombinante selon la revendication 17 pour la préparation de colza d'hiver ou de céréale d'hiver transgéniques qui provoque une surexpression combinée des gènes MADSB et FPF1.
